# EUROPÄISCHE PATENTSCHRIFT

(11) **EP 1 812 551 B1**
(45) Veröffentlichungstag und Bekanntmachung des Hinweises auf die Patenterteilung: **02.04.2008**
(21) Anmeldenummer: 05798653.1
(22) Anmeldetag: 07.11.2005
(51) Int. Cl.: C12N 1/02

(54) **VERFAHREN ZUR ABTRENNUNG VON BAKTERIEN AUS EINER FERMENTATIONSBRÜHE**
METHOD FOR REMOVING BACTERIA FROM A FERMENTATION BROTH
PROCEDE POUR ISOLER DES BACTERIES D'UN MILIEU DE FERMENTATION

(30) Priorität: 15.11.2004 CH 187004
(43) Veröffentlichungstag der Anmeldung: 01.08.2007
(73) Patentinhaber: DrM, Dr. Müller AG, 8708 Männedorf (CH); Polytag AG, 8708 Männedorf (CH)
(72) Erfinder: O'MAHONY, Kevin, 1003 Lausanne (CH); HILBRIG, Frank, 95445 Bayreuth (DE); FREITAG, Ruth, 95445 Bayreuth (DE); SCHUMACHER, Ivo, 8645 Jona (CH)
(74) Vertreter: Herrmann, Peter Johannes
(86) Internationale Anmeldenummer: PCT/CH2005/000649
(87) Internationale Veröffentlichungsnummer: WO 2006/050625

(56) Entgegenhaltungen:
- EP-A- 0 073 079
- EP-A- 0 391 253
- WO-A-01/81249
- WO-A-92/09687
- GB-A- 1 082 862
- SCHMIDT BEATA ET AL: "Polycations as displacer in high-performance bioseparation" JOURNAL OF CHROMATOGRAPHY A, Bd. 865, Nr. 1-2, 31. Dezember 2000 (2000-12-31), Seiten 27-34, XP002375243 ISSN: 0021-9673

## Beschreibung

Die vorliegende Erfindung betrifft ein Verfahren zur stufenweisen Abtrennung von Bakterien aus einer Fermentationsbrühe.

Die Anschwemmfiltration ist ein bekannter Prozess zur Abtrennung von Feststoffen aus Flüssigkeiten und Gasen. Eine besondere Bedeutung kommt ihr in biotechnischen Prozessen zu, wo Zellmaterial aus einer Kulturbrühe eines Fermenters abgetrennt werden muss.

Aus der GB 1,082,862 ist ein Verfahren zur Abtrennung von Hefezellen mittels Anschwemmfiltration bekannt. Darin werden Filterhilfsmittel wie Stärke und silikathaltige Fullererden sowie Cellite genannt. Das Verhalten der relativ grossen Hefezellen ist verschieden von demjenigen von Bakterien.

Ein Kerzenanschwemmfilter der Art ist aus der EP 0,064,795 bekannt. Das bekannte Filter wird bei der, kontinuierlichen Abtrennung von unspezifischen Abwasserschlämmen eingesetzt. Filterhilfsmittel werden nicht genannt.

Aus der WO 01/81249 A1 ist eine Vorrichtung zur Reinigung von Wasser oder Gasen von chemischen oder mikrobiologischen Verunreinigungen bekannt. Als Mikroorganismen sind Bakterien oder Viren genannt. Die bekannte Vorrichtung besteht aus einem Behälter, in welchem ein Retentionsmittel Bentonit, ein Polymer und anderes Material enthalten sein kann. Von Bentonit ist bekannt, dass es Bakterien, Viren usw. zu fixieren vermag. Deshalb wird vorgeschlagen, dass es auch Bakterien aus einem Ventilationssystem entfernen kann. Die bekannte Methode bedient sich eines Polymers. Das Bentonit ist an einem Material im Behälter fixiert. Von einer Filtration mit Filterhilfsmitteln, also einer Anschwemmfiltration kann keine Rede sein.

In der US 5,453,200 werden bei der Züchtung von Mikroorganismen diese mittels Filtration oder Zentrifugieren isoliert. Nach der Abtrennung der Mikroorganismen wird vorgeschlagen, das überstehende Filtrat mit Bentonit zu behandelt, um verunreinigende Proteine zu absorbieren. Bentonit wird in diesem Fall also nicht zur Verbesserung der Retention der Mikroorganismen eingesetzt.

Die EP 0391 253 beschreibt ein Verfahren zur Reinigung und Desodorierung von Enzymlösungen, speziell die Entfernung von störenden farblichen und geruchlichen Beimengungen unter Mithilfe von Absorptionsmitteln, unter anderem auch Bentonit. Bentonit wird nicht zur Abtrennung der Produktionsorganismen eingesetzt.

Aufgabe der Erfindung ist es, ein Verfahren zur Verfügung zu stellen, das die effiziente und möglichst vollständige Abtrennung von bakterieller Biomasse aus Fermentationen mit möglichst hohen Biomassekonzentrationen ermöglicht. Dabei ist davon auszugehen, dass die isolierten Bakterienzellen als Basismaterial für eine Weiterverarbeitung dienen.

Die Aufgabe wird erfindungsgemäss dadurch gelöst, dass in einer ersten Verfahrenstufe die bakterienhaltige Fermentationsbrühe mit einer Retentionspromotor Mischung aus einem Polykation und einem Mehrschichtsilikat inkubiert und in einer zweiten Verfahrensstufe die vorbehandelten Bakterien durch Anschwemmfiltration abgetrennt werden. Als Retentionspromotoren kommen Mischungen aus einem Polykation (Poly(ethylenimin) (PEI), Poly(diallyldimethylammoniumchlorid) (polyDADMAC)) und einem Mehrschichtsilikat (Bentonit) aber auch modifizierte (positiv-geladene) Zellulosen in Frage.

Die Konzentration an Biomasse sollte zum Zeitpunkt der Anschwemmfiltration vorzugsweise eine optische Dichte OD₆₀₀- von 15 aufweisen. Wenn nötig kann dies durch Verdünnung erreicht werden. Werden Kulturen mit höheren optischen Dichten ohne einen der Filtration vorangehenden Verdünnungsschritt filtriert, sinkt die Effizienz des Zellrückhalts und der Druckabfall über den Filterkuchen steigt stark an.

Die Anschwemmfiltration wird mit einer Durchflussrate von 500 bis 1500 l/(m2*h) durchgeführt.

Als Retentionspromotormischung hat sich die Zugabe von 5 bis 500 mg/l Polykation (PEI, PolyDADMAC) und 2 bis 10 g/l Bentonit als besonders vorteilhaft erwiesen. Unter diesen Bedingungen ist ein nahezu vollkommener Bakterienrückhalt gewährleistet (mindestens > 98 %). Bei einer Zugabe von geringeren Mengen an Polykation und/oder Bentonit sinkt die Effizienz der Bakterienretention (< 50 %); höhere Mengen an Polykationen verbessern die Filtrationseffizienz nicht weiter und führen zu höheren Filtrationsdrücken, während bei höheren Bentonitzugaben (mehr als 10 g/l) unnötig voluminöse Filterkuchen und damit unökonomische Filtrationsbedingungen erreicht werden. Gleichzeitig steigen Filtrationsdrücke ebenfalls an.

Bentonit ist ein Mehrschichtsilikat, das auch unter dem Synonymen Smektit und Montmorillonit bekannt ist.

Es hat sich als vorteilhaft erwiesen, dass mit Natrium aktiviertes Bentonit, oder Natriumbentonit, verwendet wird. Mit Natrium aktiviertes Bentonit hat besonders gute Ergebnisse geliefert. Auch Kalziumbentonit hat sich als brauchbar erwiesen.

Als Polykationen für die Retentionspromotormischung sind weiter Poly(ethylenimin) (PEI) verschiedener Hersteller hervorragend geeignet. PEI ist ein aus Ethylenimineinheiten aufgebautes Polykationen. Dabei sind hochmolekulare PEI (1000 - 50000 g/mol) besser geeignet als niedermolekulare (< 1000 g/mol), bei vergleichbarer Molmasse sind verzweigte Moleküle besser geeignet als unverzweigte. Ein zweites Polykation, das sich nachweislich gut als Retentionspromotor eignet, ist Poly(diallyldimethylammoniumchlorid) (polyDADMAC.

Während der erste Prozessschritt (Behandlung mit der Retentionspromotormischung) unter Prozessbedingungen durchgeführt wird, ist es von Vorteil, dass unmittelbar vor der Filtration und vor Zugabe des Filterhilfsmittels der pH-Wert der Suspension auf einen Wert von 8 erhöht wird, präferentiell durch Zugabe einer geeigneten Menge an Tris-Puffer.

Weiterhin ist es von Vorteil, dass unmittelbar vor der Filtration 100 -1000 mM NaCl zugemischt werden. Die Zugabe von Tris und NaCl hat den Vorteil, dass der Filtrationsdruck reduziert wird und dadurch die Durchlässigkeit des Filterkuchens leicht erhöht wird.

Bakterien sind (im Vergleich zu eukaronytischen Zellen wie Hefen, Pilzen oder Tier-/Pflanzenzellen) sehr kleine Organismen, die in der Biotechnologie in vielfältiger Weise zur Produktion eingesetzt werden. Ihre vollständige Abtrennung vom Kulturüberstand durch einfache Filtration ohne Filterhilfsmittel ist unmöglich, die Abtrennung durch Anschwemmfiltration bedingt extrem voluminöse, mechanisch stabile Filterkuchen (Einlagerung der Bakterie in den Kuchen), die im Falle der in der Bioproduktion üblichen Zelldichten um Größenordnungen höhere Filterhilfsmittelmengen bedingen würden, als gemeinhin als industriekompatibel gelten. Es ist nicht möglich die benötigten Mengen an Filterhilfsmittel durch Vorbehandlung der Bakterien (etwa durch Polykationen (e.g. PEI, PolyDADMAC), mehrfachgeladenen Metallionen (e.g. Fe3+, A13+, Ca2+), geladene Adsorbenzien wie positiv-geladene Zellulose, Quervernetzung mit negativ geladenen Silikatpartikeln) wesentlich zu reduzieren. Gleichzeitig ist in allen Fällen die Fixierung der Zellen im Filterkuchen ungenügend für den Aufbau eines robusten Prozesses. Die Bakterien werden typischerweise schon durch geringe Druck oder Volumenstromschwankungen wieder aus dem Filterkuchen ausgewaschen (ausbluten der Bakterien). Dies verunmöglicht eine Prozessierung der Bakterien im Filterkuchen (Waschen, Pufferaustausch, Zelllyse) und damit jeden Ansatz zur Prozessintensivierung durch Prozessintegration.

Die Verwendung von Bentonit und Polykationen wie PEI oder PolyDADMAC als Retentionspromotor bei der Anschwemmfiltration führt in überraschender Weise zu einer vollständigen und völlig stabilen Abtrennung der Bakterien in Filterkuchen handhabbaren Ausmaßes. Die beobachtete hohe Effizienz des Bakterienrückhaltes geht nachweislich über einen reinen Fixierungseffekt durch die Retentionspromotorenmischung hinaus und ist wahrscheinlich auf die Bildung eines Hydrogels durch das Bentonit während der Inkubationsphase zurückzuführen. In jedem Falle ist eine Bakterienfiltration nach Behandlung der Kultur mit PEI (polyDADMAC)/Bentonit von grundlegend anderer Qualität (Druckabfall, Filtrierbarkeit, Bakterienrückhalt, Fixierung der Bakterien im Filterkuchen) als eine anderweitig analoge Filtration (Filterhilfsmittel, Durchflussgeschwindigkeit) ohne Vorbehandlung oder nach Vorbehandlung mit einem Polykation allein.

### Beispiel für Bakterien

Bakterien des Stammes (E. Coli DH5 von Clontech-BD Biosciences, Palo Alto, California, USA) wurden in bekannter Weise in einem Bioreaktor (Chemap FZ) kultiviert. Das Kultur-Medium (pH 6.8) bestand aus wenigstens 10 g/l Glucose, 2 g/L NaCl, 1.2 g/l MgSO4, 6 g/l Na2HPO4, 3g /l KH2PO4, 3 gll (NH4)2SO4, 1.7 g/l Zitronensäure, 0.01 g/l CaCl2 und Spurenelementen (FeCl3 60 mg/l, Na2EDTA 8.4 mg/l, Zn(CH3COO)2 8.0 mg/l, MnCl2 15.0 mg/l, CoCl2 2.5 mg/l, Na2MoO4 2.5 mg/l, CuCl2 1.5 mg/l, H3B03 3.0 mg/l.). 02 wurde nicht gemessen, die Kultivierung jedoch bei maximalem Luftstrom, d. h. etwa 30 l/min, durchgeführt. Eine Temperatur von 37 °C wurde eingestellt. Nach 16 Stunden war der Biorektor bereit zur Ernte (OD600 > 15).

Die Filtration erfolgte mittels eines Laborkerzenfilters (Type TSD-0.012m2 der Firma DrM, Dr. Müller AG, Männedorf, Schweiz). Der Laborkerzenfilter hat eine Oberfläche von 75 cm2 oder 40 cm2 und erlaubt je nach Biomassekonzentration die Filtration von einigen Litern Zellsuspension pro Ansatz. Eine Membran (B31M30, G11M200, oder G11M080, alles geliefert von DrM) unterstützt von einer Metallplatte wurde als Filtermittel verwendet. Verwendet wurden verschiedene Filterhilfsmittel (Diatomeenerden), unter anderem die "Celpure"-Materialien der Firma, World Minerals Inc. (Advanced Minerals and Celite), St Barbara, CA, USA und die "Celatom"-Materialien der Firma Eagle Picher Filtration and Minerals Europe.

Die Versuche wurden unter industrietypischen Bedingungen durchgeführt. Die Durchflussrate wurde zwischen 500 und 1500 l/(m2*h) eingestellt. Die Menge an Filterhilfsmittel betrug zwischen 10 und 20 g/l. Alle Versuche wurden - soweit nicht gesondert gekennzeichnet - mit Bakterienkulturen E. Coli der nominellen OD600 von 15 durchgeführt.

OD = Optische Dichte. Die Messung wird in bekannter Weise mit Licht der Wellenlänge 600nm durchgeführt und gibt die Trübung der Lösung an. Sie gibt den Messwert der Konzentration der Bakterien in der Suspension an.

Versuche mit verschiedenen Arten und Körnungen sowie Mengen von Filterhilfsmitteln wie Kieselguren (Diatomeenerden) führten nicht zum Erfolg. Lediglich unwirtschaftlich grosse Mengen an Kieselguren brachten eine brauchbare Klärung. Eine wirksame und wirtschaftliche Abtrennung der Bakterien einer Kultur ist nur mit Filterhilfsmitteln nicht möglich. Darüber hinaus werden die Bakterien nicht ausreichend stabil im Filterkuchen zurückgehalten. Das macht die Filtration unsicher und beeinträchtigt bzw. verunmöglicht eine weitere Behandlung (Waschen, Zelllyse) der bakterienhaltigen Filterkuchen.

Versuchsergebnisse sind in den Tabellen 1 und 2 aufgezeigt:

**Table 1 Results of the body feed filtration experiments with various diatomaceous earths as filter aids.**

| **Amount and type of filter aid** | **Final pressure** | **Bacteria retention*** |
|---|---|---|
| 5 g/L Celpure P100 | 3 bar** | 72 % |
| 10 g/L Celpure P100 | 3 bar** | 65 % |
| 5 g/L Celpure P300 | 2.85 bar** | 36 % |
| 10 g/L Celpure P300 | 0.8 bar | 38.5 % |
| 15 g/L Celpure P300 | 0.6 bar | 50 % |
| 20 g/L Celpure P300 | 0.8 bar | 74 % |
| 30 g/L Celpure P300 | 0.4 bar | 75 % |
| 0.25 g/L Celpure P 1000 | 1.8 bar | 13 % |
| 1 g/L Celpure P1000 | 0.6 bar | 16 % |
| 5 g/L Celpure P 1000 | 0 bar | 19 % |
| 10 g/L Celatom FW 12 | 0.9 bar | 69 % |
| 30 g/L Celatom FP4 | 1.35 | 66 % |

| | | |
|---|---|---|
| * always refers to the amount retained in the cake in regard to the volume, ** the filtration had to be terminated prematurely due to clogging of the filter cake. | | |

**Table 2 Results of the body feed filtration experiments with mixtures of fine and coarse diatomaceous earths as filter aids.**

| **Ratio P1000 : P100, total amount** | **Final pressure** | **Bacteria retention** |
|---|---|---|
| P 1000 alone (5 g/L) | 0.05 bar | 23 % |
| 9 : 1,2 g/L | 2.6 bar* | 9% |
| 9 : 1,5 g/L | 1.1 bar | 23 % |
| 9 : 1,10 g/L | 0.3 bar | 23 % |
| 9 : 1,15 g/L | 0.2 bar | 39 % |
| 3 : 1,2 g/l | 2.5 bar* | 19 % |
| 3 : 1,5 g/L | 1.2 bar | 27% |
| 3 : 1,10 g/L | 0.4 bar | 33 % |
| 3 : 1,15 g/l | 0.2 bar | 43% |
| 1 : 1,5 g/L | 1.5 bar | 53% |
| 1 : 1,10 g/L, | 0.6 bar | 37 % |
| 1 : 4,5 g/L | 3.0 bar* | 55 %. |
| 1 : 4, 10 g/L | 1.5 bar | 62 % |
| Celatom FW6 : FW12, 1:1 10g/L | 2.4 bar* | 82 % |

| | | |
|---|---|---|
| * filtration had to be terminated prematurely due to clogging of the filter cake. | | |

Auch Versuche zu Filtrationen mittels anderer Filterhilfsmittel (Zellulose, J. Rettenmaier & Söhne, Rosenberg, Deutschland, keinerlei Retention zu beobachten) oder nach Flockulierung der Bakterien durch Polykationen (PEI, PolyDADMAC) oder mehrfach positiv geladene Metallionen (Fe3+, A13+, Ca2+), Tabelle 3, bzw. Adsorption an positiv-geladener Cellulose, Tabelle 4, und Quervernetzung durch positiv-geladene Ionen (Polykationen, Metallionen) mit negativ geladenen Filterhilfsmittelpartikein, Tabelle 5, waren erfolglos im Sinne eines möglichst vollständigen, aber vor allem stabilen Rückhalts der Bakterien in einem Filterkuchen industriekompatiblen Ausmasses Es ist allerdings anzumerken, dass die auf positiv-geladener Zellulose (ebenfalls J. Rettenmaler & Söhne) adsorbierten Bakterien (Retention in der Regel < 50 % bei den hier berücksichtigten "Industrie-kompatiblen" maximalen Filterhilfsmittelmengen) nach der Filtration stabil im Filterkuchen etabliert waren und solche Filterkuchen z.B. ohne merkliches Ausbluten der Bakterien mit verschiedenen Waschpuffern gewaschen werden konnten. Dies wurde bei keinem anderen der bislang dargelegten Experimente beobachtet.

**Table 3 Results of the filtration experiments after flocculation with PEI.**

| **Conditions** | **Final pressure** | **Bacteria retention** |
|---|---|---|
| FW 6 10 g/L, PEI 3 50 mg/L | 1.6 bar | 81 % |
| FW 12 10g/L, PEI 3 50mg/L | 1.1 bar | 77 % |
| FW 12 10g/L, PEI 3 25mg/L, pH 8, 50 mM NaCl | 3.0 bar | 86% |
| FW 12 20g/L, PEI 3 25mg/L, pH 8, 50 mM NaCl | 1.7 bar | 87 % |
| FW12 20g/L, PEI 3 37.5mg/L, pH 8, 50 mM NaCl | 1.5bar | 87.5 % |
| FW 12 10 g/L, PEI4 5 mg/L | 2.0 bar | 87 % |
| FW 12 10 g/L, PEI4 25 mg/L | 1.4 bar | 94 % |
| FW 12 10 g/L, PEI4 150 mg/L | 2.0 bar | 100 % |
| | | |
| FW 14 10g/L, PEI 3 25mg/L, pH 8, NaCl 50 mM | 2.2 bar | 83 % |
| FW14 20g/L, PEI 3 37.5mg/L, pH 8 | 1.5 bar | 87 % |
| FW 14 20g/L, PEI 3 37.5mg/L, pH 8, NaCl50 mM | 1.2 bar | 86 % |
| | | |
| FW20 10g/L, PEI 3 25 mg/L, pH 8, NaCl 50 mM | 1.5 bar | 74 % |
| FW20 10g/L, PEI4 50 mg/L | 3.0 bar | 70 % |
| FW 20 20 g/L, PEI 4 50 mg/L | 2.6 bar | 92 % |

| | | |
|---|---|---|
| * filtration had to be terminated prematurely due to clogging ofthe filter cake. | | |

**Table 4 Capture/sedimentation of bacteria with charged cellulose.**

| **Conditions** | **BE600/20 10 g/L** | **BE600/20 5 g/L** | **BE600/20 1 g/L** | **FDY 600 10 g/L** | **FIC 200 10 g/L** | **EFC 900 CT 10 g/L** | **EFC 950 C 10 g/L** |
|---|---|---|---|---|---|---|---|
| pH 5.5 | | | | | | | |
| 0.0 M NaCl | 31 % | | | 27 % | 28 % | 23 % | 24 % |
| 0.1 M NaCl | 23 % | 17 % | 12 % | | | | |
| 0.25 M | 23.5% | 18 %, | 11% | | | | |
| NaCl | | | | | | | |
| 0.5 M NaCl | 28 % | 22.5 % | 13% | | | | |
| | | | | | | | |
| pH 7.0 | | | | | | | |
| 0.0 M NaCl | 29 % | | | 20 % | 27 % | 21 % | 18 % |
| 0.1 M NaCl | 25 % | 22 % | 18 % | | | | |
| 0.25 M | 30 % | 23 % | 16 % | | | | |
| NaCl | | | | | | | |
| 0.5 M NaCl | 28 % | 17 % | 18% | | | | |
| | | | | | | | |
| pH 8.0 | | | | | | | |
| 0.0 M NaCl | 29 % | | | 17% | 24.5 % | 20 % | 17 % |
| 0.1 M NaCl | 36 % | 34 % | 28% | | | | |
| 0.25 M | 38.5% | 29% | 27% | | | | |
| NaCl | | | | | | | |
| 0.5 M NaCl | 37 % | 29 % | 28 % | | | | |

**Table 5 Filtration of bacteria cross linked to diatomaceous earth**

| **Conditions** | **Final pressure** | **Bacteria retention** |
|---|---|---|
| 10g/L FW 12, 10 mg/L AlCl₃ | 3.0 bar* | 75.5 % |
| 10g/L FW 12, 1 mg/L AlCl₃ | 3.5 bar | 74 % |
| 10 g/L FW 12, 0.1 mg/L AlCl₃ | 2.0 bar | 69 % |
| | | |
| 10 g/L FW 12, 5 mg/L PEI4 | 2.0 bar* | 87 % |
| 10 g/L FW 12, 25 mg/L PEI4 | 1.4 bar* | 94 % |
| 10 g/L FW 12, 150 mg/L PEI4 | 2.0 bar* | 100 % |
| 10g/L P1000, 75 mg/L PEI 4. | 1.1 bar | 88 % |
| 10. g/L, P 1000, 100 mg/L PEI4 | 1.3 bar | 97 % |
| 10 g/L P1000, 125 mg/L PEI4 | 1.2 bar | 95 % |
| 10 g/L FW20, 50 mg/L PEI4 | 3.0 bar | 70 % |
| 20 g/L FW 20, 50 mg/L PEI 4 | 2.6 bar | 92 % |
| 10 g/L FW12, 10 g/L P1000, 5 mg/L PEI4 | 0.6 bar | 75 % |
| 10 g/L FW 12, 10 g/L P 1000, 25 mg/L PEI4 | 0.4 bar | 83 % |
| 10 g/L FW12, 10 g/L P1000, 150 mg/L PEI4 | 1.5 bar | 90.5 % |

| | | |
|---|---|---|
| * filtration had to be terminated prematurely due to clogging of the filter cake. | | |

### Versuche mit Bentonit:

Bentonit ist ein Mehrschichtsilikat, das in Kontakt mit wässerigen Lösungen mehr oder weniger stark aufquillt. Diese Quellung führen zu einer Absorption von unterschiedlichen Stoffen aus der Umgebung, so auch Bakterien. Nach zahlreichen Vorversuchen wurde mit Natrium aktiviertes Bentonit zur Durchführung des Ausführungsbeispiels gewählt. Das Mineral quillt in einem Ausmass das zwischen Natriumbentonit, welches stark quillt, und Kalziumbentonit, das sehr wenig quillt, liegt. Das Mineral weist positive Ladungen in den Räumen der Zwischenschichten auf. Dies erlaubt eine elektrostatische Wechselwirkung mit den negativ geladenen Bakterien. Das Mineral hat daher theoretisch die Fähigkeit Bakterien sowohl durch Adsorption als auch durch Absorption zu binden.

Die Ergebnisse von zahlreichen Versuchen zur Anschwemmfiltration von Bentonit behandelten Bakterien sind in Tabelle 6 zusammengefasst. Dabei wurden sowohl Versuche zur Vorbehandlung der Bakteriensuspension mit Bentonit allein, als auch solche bei denen neben Bentonit noch mehrfach geladene Metallionen oder Polykationen zur Vorbehandlung verwendet wurden, berücksichtigt. Während eine Behandlung mit Bentonit allein, insbesondere bei Kulturen mit geringer Biomasse (OD600 < 15) und Minimalmedien (LB-Medium), gelegentlich zum Erfolg führte, konnte ein robuster und zuverlässiger Prozess nur durch die Behandlung (Inkubation) mit der Bentonit/Polykation-Mischung aufgebaut werden.

**Table 6, Filtration of bacteria suspension in the presence of Bentonit.**

| **Conditions** | **Final pressure** | **Bacteria retention** |
|---|---|---|
| 20 g/L FW 12, 5 g/L WGA, LB-medium* | 1.4 bar | 93 % |
| 20 g/L FW 14,5 g/L WGA, LB-medium* | 3 bar | - |
| 20 g/L FW 14,5 g/L WGA, LB-medium* | 1.3 bar | - |
| 20 g/L FW 14,5 g/L WGA, LB-medium, 250 mM NaCl. | 0.2 bar | 52 % |
| 20 g/L FW 14,5 g/L WGA, LB-medium, 500 mM NaCl | 0.3 bar | 53 % |
| 20 g/L FW 14, 5 g/L WGA, LB-medium, 50 mM Tris pH 8, 250 mM NaCl | 0.5 bar | 55 % |
| 20 g/L FW 50, 5 g/L CV, LB-medium* | 1.3 bar | 94 % |
| | | |
| 20 g/L FW 14, 5 g/L WGA, 50 mM Tris pH 8, 250 mM NaCl, 0.1 g/L FeCl₃ | 0.4 bar | 69% |
| 20 g/L FW 14, 5 g/L WGA, 50 mM Tris pH 8, 250 mM NaCl, 0.2 g/L FeCl₃ | 1.4 bar | 89 % |
| 20 g/L FW 14, 5 g/L WGA, 50 mM Tris pH 8, 250 mM NaCl, 0.3 g/L FeCl₃ | 1.8 bar | 93 % |
| 20 g/L FW 14, 5 g/L WGA, 50 mM Tris pH 8, 250 mM NaCl, 0.4 g/L FeCl₃ | 2.2 bar | 92 % |
| | | |
| 20 g/L FW 14, 5 g/L WGA, 50 mM NaCl, 50 mg/L PEI4 | 0.4 bar | 65 % |
| 20 g/L FW 14, 5 g/L WGA, 50 mM Tris pH 8, 50mg/L PEI4 | 2.1 bar | 100 % |
| 20 g/L FW 14, 5 g/L WGA, 50 mM Tris pH 8, 250 mM NaCl, 50 mg/L PEI4 | 1.4 bar | 100 % |
| 20 g/L FW 14, 5 g/L WGA, 50 mM Tris pH 8, 1000 mM NaCl, 50 mg/L PEI4 | 0.6 bar | 98 % |
| 20 g/L FW 14, 5 g/L WGA, 50 mM Tris pH 8, 250 mM NaCl, 100 mg/L PEI4 | 0.6 bar | 100 % |
| 20 g/L FW 14, 5 g/L WGA, 50 mM Tris pH 8, 250 mM NaCl, 50 mg/L polyDADMAC | 0.2 bar | 58 % |
| 20 g/L FW 14, 5 g/L WGA, 50 mM Tris pH 8, 250 mM NaCl, 150 mg/L polyDADMAC | 0.9 bar | 100 % |
| | | |
| 20 g/L FW 14, 5 g/L WGA, 50 mM Tris pH 8, 250 mM NaCl, 50 mg/L PEI4 | 0.9 bar | 100 % |
| 20 g/L FW 14, 5 g/L WGA, 50 mM Tris pH 8, 250 mM NaCl, 50 mg/L PEI4* * | 3 bar | 100 % |

| | | |
|---|---|---|
| *filtration terminated prematurely due to clogging of the filter cake, ** repetition of the experiment above with a culture containing much debris | | |

Die Filtration der auf diese Weise fixierten Bakterien führte überraschenderweise zu einer nahezu 100%-igen Retention. Ein Auswaschen der Bakterien (erkenntlich durch eine deutlich sichtbare Zunahme der Trübung des Filtrats) ist selbst nach Aufgabe eines Druckpulses bei solchen Filtrationen nicht zu beobachten.

Die Wirksamkeit des Bentonit lässt sich nicht allein mit Adsorption/Absorption erklären. Es tritt ein Synergieeffekt auf, der die Rückhaltung der Bakterien offensichtlich verbessert. Wahrscheinlich ist dieser Effekt auf die Ausbildung von Hydrogelen durch das Bentonit verursacht. Beste Ergebnisse werden bei pH 8 beobachtet, wahrscheinlich, weil hier die positive Ladung des PEI stark genug ausgeprägt ist für eine stabile Wechselwirkung mit den negativ geladenen Bakterien. Bei Versuchen ohne Polykation-Zusatz konnte eine Absenkung des pH-Wertes auf Werte zwischen 5.0 und 5.5 das Verfahren im Sinne einer Senkung des unter sonst vergleichbaren Bedingungen benötigten Filtrationsdrucks verbessern. Dies ist wahrscheinlich auf einen Abbau der pHsensitiven Hydrogele zurückzuführen. Damit überein stimmt auch die Beobachtung, das solche Suspensionen nach Senkung des pH-Wertes möglichst rasch weiterverarbeitet werden müssen, da sonst sowohl die Bakterienretention als auch die Stabilität der Bakterien im Filterkuchen abnehmen. Auch eine Zugabe von bis zu 1 M NaCl wirkt sich verringernd auf den zur Filtration notwendigen Druck aus, ohne die Bakterienretention/Stabilität merklich zu verschlechtern.

Insgesamt sind diese Effekte jedoch vor allem bei den Filtrationen ohne Polykation-Zusatz zu beobachten. Werden die Bakterien nach Vorbehandlung mit Bentonit/Polykation bei pH 8 filtriert ist der Prozess sehr robust und andere Parameter wie Temperatur, Salzgehalt oder Medienzusammensetzung spielen kaum noch eine Rolle für den Erfolg.

### Ausführungsbeispiel:

Am Ende der Bakterienkultur wird der Inhalt im Bioreaktor (Fermenter) mit 5 g/l Bentonit und 50 mg/l PEI versetzt und bei Raumtemperatur 1 Stunde lang durchmischt. Anschließend wird die Bakteriensuspension durch Zugabe einer geeigneten Tris-gepufferten Lösung auf eine nominelle Biomassenkonzentration von nicht mehr als OD600 = 15, einen pH-Wert von 8, eine NaCl-Konzentration von 250 mM und eine Tris-Konzentration von 50 mM gebracht. Dann wird das Filterhilfsmittel zugegeben (e.g. 20 g/l Celatom FW 14) und die Mischung zügig bei einer Durchflussrate von 600 1.(m2*h) filtriert. Es wurden eine Filtrationen mit und ohne Zugabe von Bentonit durchgeführt.
Die Mittelwerte der Ergebnisse sind graphisch darstellt. Es zeigen:
- Fig. 1: Einen Vergleich der Filtration mit und ohne Bentonit.
- Fig. 2: Die Rückhaltekapazität der Bakterien im Filterkuchen mit und ohne Bentonit.

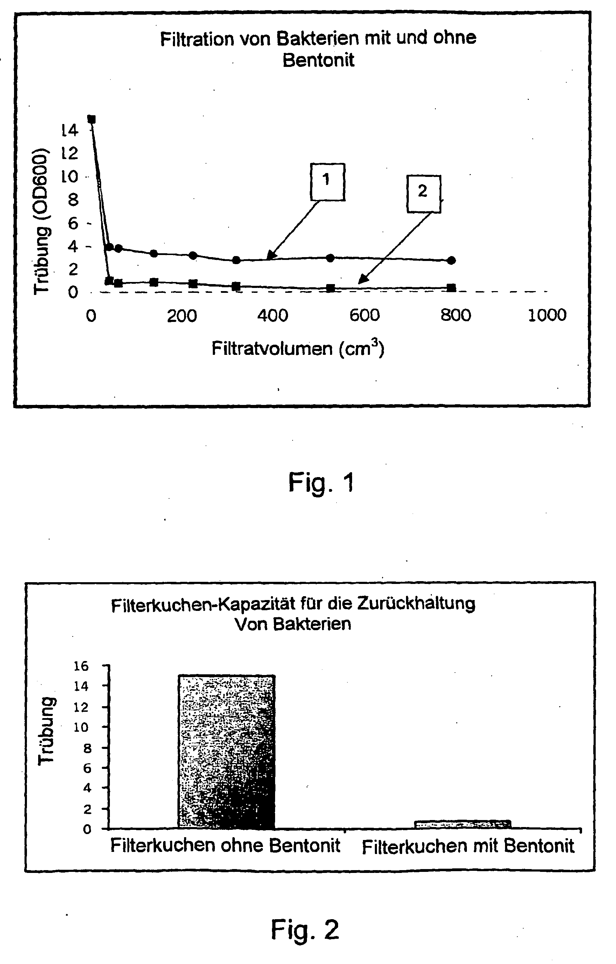

In Figur 1 zeigt Kurve 1 die Filtration ohne Bentonit; Kurve 2 die Filtration unter Zugabe von Bentonit, wobei überraschenderweise eine 95 - 100-%-ige Retention der Bakterien, sichtbar an einer gegen Null gehenden Trübung ersichtlich ist.

Die Filtration unter Verwendung von Bentonit ergibt ein klares Filtrat, abhängig vom Typ des verwendeten Bentonit. Um den gleichen Effekt mit bekannten Filterhilfsmitteln allein zu erreichen wären um eine Grössenordnung höhere Mengen an Filterhilfsmittel erforderlich.

Figur 2 zeigt die Ergebnisse nach einer Auswaschung des Filterkuchens. Ein unter hohem Druck auf den Filterkuchen ohne Bentonit aufgegebener Waschpuffer würde zu einer Ablösung der Bakterien vom Kuchen führen (linke Säule). Sie sind nicht fest mit dem Kuchen verbunden. Ein Filterkuchen mit Bentonit hingegen, kann ohne Bakterienverlust gewaschen werden (rechte Säule). Daraus ergibt sich, dass der mit Bentonit erhaltene Filterkuchen von wesentlich besserer Qualität ist.

Ein grosser Vorteil des erfindungsgemässen Verfahrens ist, dass es ausgezeichnet zur Behandlung grosser Bakterienmengen geeignet ist, da bei Verwendung von Bentonit ein permeabler, stabiler Filterkuchen erzielt wird. Der Filterkuchen kann problemlos mit anderen Flüssigkeiten gewaschen oder weiteren Prozessschritten wie einer In-Situ-Zelllyse unterworfen werden. Ausserdem kann ein solcher Filterkuchen auch leicht mittels Luft getrocknet werden. Weiter kann der Filterkuchen am Ende der Filtration leicht von den Filterelementen, beispielsweise eines Kerzenfilters, entfernt werden. Es gibt kein Kleben an den Filtermitteln.

## Patentansprüche

1. Verfahren zur stufenweisen Abtrennung von Bakterien aus einer Fermentationsbrühe, **dadurch gekennzeichnet, dass** in einer ersten Verfahrenstufe die Bakterien in der Fermentationsbrühe mit einer Retentionspromotor-Mischung aus einem Polykation und einem Mehrschichtsilikat inkubiert und in einer zweiten Verfahrensstufe die vorbehandelten Bakterien durch Anschwemmfiltration abgetrennt werden.

2. Verfahren nach Anspruch 1, **dadurch gekennzeichnet, dass** die Anschwemmfiltration mit einer Durchflussrate von 500 bis 1500 I/(m2*h) durchgeführt wird.

3. Verfahren nach Anspruch 1, **dadurch gekennzeichnet, dass** für die Anschwemmfiltration zwischen 2 und 20 g/l an Filterhilfsmittel verwendet Werden.

4. Verfahren nach Anspruch 3, **dadurch gekennzeichnet, dass** die Anschwemmfiltration mit Kieselguren (Diatomeenerden) als Filterhilfsmittel durchgeführt wird.

5. Verfahren nach Anspruch 1, **dadurch gekennzeichnet, dass** die Fermentationsbrühe für eine Stunde mit der Retentionspromotormischung inkubiert wird.

6. Verfahren nach Anspruch 1, **dadurch gekennzeichnet, dass** in der Retentionspromotormischung 2 bis 10 g/l Bentonit verwendet werden.

7. Verfahren nach Anspruch 6, **dadurch gekennzeichnet, dass** als Bentonit Natriumbentonit verwendet wird.

8. Verfahren nach Anspruch 6, **dadurch gekennzeichnet, dass** als Bentonit Kalziumbentonit verwendet wird.

9. Verfahren nach Anspruch 1, **dadurch gekennzeichnet, dass** in der Retentionspromotomnischung 0.5 bis 500 mg/l PEI verwendet werden.

10. Verfahren nach Anspruch 9, **dadurch gekennzeichnet, dass** in der Retentionspromotormischung PEI mit einer Molmasse zwischen 1000 und 50000 g/mol verwendet wird.

11. Verfahren nach Anspruch 9, **dadurch gekennzeichnet, dass** in der Retentionspromotormischung verzweigtes PEI verwendet wird.

12. Verfahren nach Anspruch 1, **dadurch gekennzeichnet, dass** in der Retentionspromotormischung 0.5 bis 500 mg/l polyDADMAC verwendet werden.

13. Verfahren nach Anspruch 1, **dadurch gekennzeichnet, dass** unmittelbar vor der Filtration der pH-Wert der Suspension auf den Wert 8 eingestellt wird.

14. Verfahren nach Anspruch 1, **dadurch gekennzeichnet, dass** unmittelbar vor der Filtration 50 -1000 mM NaCl zugemischt werden.

15. Verfahren nach Anspruch 1, **dadurch gekennzeichnet, dass** unmittelbar vor der Filtration eine Pufferkonzentration von 50 mM eingestellt wird.

16. Verfahren nach Anspruch 1, **dadurch gekennzeichnet, dass** unmittelbar vor der Filtration eine Pufferkonzentration von 50 mM Tris eingestellt wird.

17. Verfahren nach Anspruch 1, **dadurch gekennzeichnet, dass** die Konzentration der Bakterien in der Fermentationsbrühe eine optische Dichte OD600 von höchstens 15 aufweist.

18. Verfahren nach Anspruch 1, **dadurch gekennzeichnet, dass** die nominelle Konzentration der Bakterien in der Fermentationsbrühe nach der Inkubation mit dem Retentionspromotor und unmittelbar vor der Filtration auf einen Wert von OD600 = 15 eingestellt (verdünnt) wird.

19. Verfahren nach Anspruch 1, **dadurch gekennzeichnet, dass** die nominelle OD600 zusammen mit dem pH-Wert, der NaCl- und der Pufferkonzentration durch eine geeignete Tris-gepufferte Lösung eingestellt wird.

20. Verfahren nach Anspruch 1, **dadurch gekennzeichnet, dass** als Retentionspromotor modifizierte (positiv-geladene) Zellulosen verwendet werden:

## Claims

1. Process for stepwise removal of bacteria from a fermentation broth, **characterized in that** in a first processing step the bacteria of the fermentation broth are incubated with a retention-promoting mixture, consisting of a polycation and a multiplayer silicate, and in the second processing step the pretreated bacteria are removed by precoat filtration.

2. Process as defined in claim 1, **characterized in that** the precoat filtration is carried out at a flow rate of 500 to 1500 I/(m²*h).

3. Process as defined in claim 1, **characterized in that** between 2 and 20 g/L of filter aid is used for the precoat filtration.

4. Process as defined in claim 3 , **characterized in that** the precoat filtration is carried out with a kieselguhr (diatomaceous earth) as filter aid.

5. Process as defined in claim 1, **characterized in that** the fermentation broth is incubated with the retention-promoting mixture for one hour.

6. Process as defined in claim 1, **characterized in that** the retention-promoting mixture contains from 2 to 20 g/L of bentonite.

7. Process as defined in claim 6, **characterized in that** the bentonite is sodium bentonite.

8. Process as defined in claim 6, **characterized in that** the bentonite is calcium bentonite.

9. Process as defined in claim 1, **characterized in that** the retention-promoting mixture contains from 0.5 to 500 mg/L of PEI.

10. Process as defined in claim 9, **characterized in that** the PEI contained in the retention-promoting mixture has a molecular weight between 1000 and 50,000 g/mol.

11. Process as defined in claim 9, **characterized in that** the retention-promoting mixture contains a branched PEI.

12. Process as defined in claim 1, **characterized in that** the retention-promoting mixture contains from 0.5 to 500 mg/L of polyDADMAC.

13. Process as defined in claim 1, **characterized in that** just before the filtration the pH of the suspension is adjusted to a value of 8.

14. Process as defined in claim 1, **characterized in that** just before the filtration 50-1000 mM of NaCl is mixed in.

15. Process as defined in claim 1, **characterized in that** just before the filtration the buffer concentration is adjusted to 50 mM.

16. Process as defined in claim 1, **characterized in that** just before the filtration the buffer concentration is adjusted to 50 mM tris.

17. Process as defined in claim 1, **characterized in that** the concentration of the bacteria in the fermentation broth has an optical density OD₆₀₀ of at the most 15.

18. Process as defined in claim 1, **characterized in that** the nominal concentration of the bacteria in the fermentation broth after the incubation with the retention promoter and just before the filtration is adjusted (diluted) to a value of OD₆₀₀ = 15.

19. Process as defined in claim 1, **characterized in that** the nominal OD₆₀₀, the pH, the NaCl concentration and the buffer concentration together are adjusted with a suitable tris-buffered solution.

20. Process as defined in claim 1, **characterized in that** the retention promoter used is a modified (positively charged) cellulose.

## Revendications

1. Procédé pour isoler par étapes des bactéries d'un milieu de fermentation, **caractérisé en ce que** dans une première étape du procédé, les bactéries dans le milieu de fermentation sont incubées avec un mélange de promoteurs de rétention constitué par un polycation et un silicate multicouche et que dans une deuxième étape du procédé, les bactéries préalablement traitées sont isolées par filtration sur précouches.

2. Procédé selon la revendication 1, **caractérisé en ce que** la filtration sur précouches est réalisée avec un débit de 500 à 1500 1/(m2*h).

3. Procédé selon la revendication 1, **caractérisé en ce qu'**on utilise entre 2 et 20 g/l d'auxiliaire de filtration pour la filtration sur précouches.

4. Procédé selon la revendication 3, **caractérisé en ce qu'**on réalise la filtration sur précouches avec du kieselguhr (terres d'infusoires) en tant qu'auxiliaire de filtration.

5. Procédé selon la revendication 1, **caractérisé en ce que** le milieu de fermentation est incubé pendant 1 heure avec le mélange de promoteurs de rétention.

6. Procédé selon la revendication 1, **caractérisé en ce qu'**on utilise 2 à 10 g/l de bentonite dans le mélange de promoteurs de rétention.

7. Procédé selon la revendication 6, **caractérisé en ce qu'**on utilise de la bentonite de sodium en tant que bentonite.

8. Procédé selon la revendication 6, **caractérisé en ce qu'**on utilise de la bentonite de calcium en tant que bentonite.

9. Procédé selon la revendication 1, **caractérisé en ce qu'**on utilise 0,5 à 500 mg/l de PEI dans le mélange de promoteurs de rétention.

10. Procédé selon la revendication 9, **caractérisé en ce qu'**on utilise dans le mélange de promoteurs de rétention, une PEI ayant une masse molaire comprise entre 1000 et 50 000 g/mole.

11. Procédé selon la revendication 9, **caractérisé en ce qu'**on utilise de la PEI ramifiée dans le mélange de promoteurs de rétention.

12. Procédé selon la revendication 1, **caractérisé en ce qu'**on utilise 0,5 à 500 mg/l de polyDADMAC dans le mélange de promoteurs de rétention.

13. Procédé selon la revendication 1, **caractérisé en ce qu'**on règle la valeur de pH de la suspension à 8 directement avant la filtration.

14. Procédé selon la revendication 1, **caractérisé en ce qu'**on ajoute un mélange 50 à 1000 mM de NaCl directement avant la filtration.

15. Procédé selon la revendication 1, **caractérisé en ce qu'**on règle une concentration de tampon de 50 mM directement avant la filtration.

16. Procédé selon la revendication 1, **caractérisé en ce qu'**on règle une concentration de tampon de 50 mM de Tris directement avant la filtration.

17. Procédé selon la revendication 1, **caractérisé en ce que** la concentration des bactéries dans le milieu de fermentation présente une densité optique D0600 d'au plus 15.

18. Procédé selon la revendication 1, **caractérisé en ce que** la concentration nominale des bactéries dans le milieu de fermentation après l'incubation avec le promoteur de rétention et directement avant la filtration est réglée (diluée) à une valeur D0600 de 15.

19. Procédé selon la revendication 1, **caractérisé en ce que** la D0600 nominale est réglée conjointement avec la valeur de pH, la concentration de NaCl et la concentration de tampon par une solution tamponnée au Tris appropriée.

20. Procédé selon la revendication 1, **caractérisé en ce qu'**on utilise des celluloses modifiées (chargées positivement) en tant que promoteur de rétention.
